(19) Europäisches Patentamt
European Patent Office
Office européen des brevets

(11) Veröffentlichungsnummer: **0 226 199**
**A2**

# (12) EUROPÄISCHE PATENTANMELDUNG

(21) Anmeldenummer: 86117361.5

(22) Anmeldetag: 12.12.86

(51) Int. Cl.[4]: **A61K 31/40**, A61K 31/42, A61K 31/425, A61K 31/44, A61K 31/54, A61K 31/535

(30) Priorität: 12.07.86 DE 3623681
20.12.85 DE 3545206

(43) Veröffentlichungstag der Anmeldung: 24.06.87 Patentblatt 87/26

(84) Benannte Vertragsstaaten: AT BE CH DE ES FR GB IT LI NL SE

(71) Anmelder: **Beiersdorf Aktiengesellschaft**
Unnastrasse 48
D-2000 Hamburg 20(DE)

(72) Erfinder: **Cohnen, Erich, Dr.**
Moorende 74
D-2155 Jork(DE)

## (54) Isoindol-Derivate als Arzneimittel.

(57) Die Isoindolin-Derivate der Formel I,

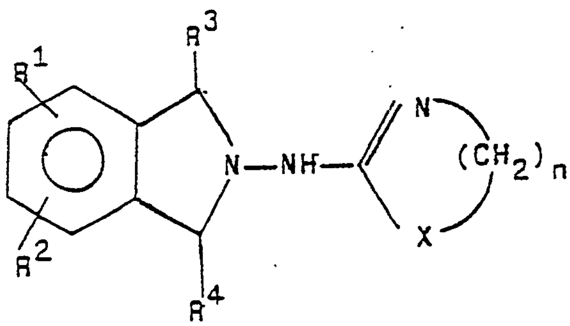

(I)

in der $R^1$ und $R^2$, die gleich oder verschieden sein können, ein Wasserstoff-oder Halogenatom, eine Alkylgruppe oder Alkoxygruppe mit jeweils 1 -4 Kohlenstoffatomen, $R^3$ und $R^4$, die gleich oder verschieden sein können, ein Wasserstoffatom oder eine Alkylgruppe mit 1 -4 Kohlenstoffatomen,
n die Zahl 2 oder 3 und
X ein Sauerstoff-oder Schwefelatom, eine Methylengruppe, eine Imino-oder Acyliminogruppe bedeuten und deren pharmazeutisch verträgliche Säureadditionssalze können zur Behandlung und/oder Prophylaxe von Migräne, Angina pectoris, Myokardinfarkt, Asthma, koronaren und peripheren Durchblutungsstörungen, beispielsweise Morbus Raynaud, thromboembolischen Erkrankungen, Diabetes, Fettsucht und Glaukom sowie Lungen-, Bronchial-und Coronarerkrankungen, metabolischen Störungen und ischämiebedingten Arrhythmien verwendet werden.

EP 0 226 199 A2

## Arzneimittel, Verfahren zu ihrer Herstellung und Behandlungsverfahren

Die vorliegende Erfindung bezieht sich auf neue Arzneimittel, Verfahren zu ihrer Herstellung und neue Behandlungsverfahren.

In den deutschen Offenlegungsschriften 28 16 627 und 29 05 501 sowie der europäischen Patentanmeldung 72 954 sind substituierte Isoindoline mit blutdrucksenkender und blutdrucksteigernder Wirkung beschrieben.

Es wurde nun festgestellt, daß neue und bestimmte, dort in allgemeiner Form beschriebene oder in den oben genannten Veröffentlichungen genannte Verbindungen überraschenderweise auch andere Wirkungen bei Säugetieren, den Menschen eingeschlossen, aufweisen und daher auch zur Behandlung und oder zur Prophylaxe weiterer Krankheiten geeignet sind.

Die Isoindolin-Derivate der Formel I,

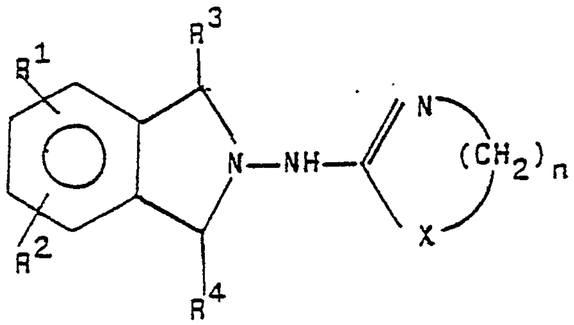

(I)

in der

$R^1$ und $R^2$, die gleich oder verschieden sein können, ein Wasserstoff-oder Halogenatom, eine Alkylgruppe oder Alkoxygruppe mit jeweils 1 -4 Kohlenstoffatomen,

$R^3$ und $R^4$, die gleich oder verschieden sein können, ein Wasserstoffatom oder eine Alkylgruppe mit 1 -4 Kohlenstoffatomen,

n die Zahl 2 oder 3 und

X ein Sauerstoff-oder Schwefelatom, eine Methylengruppe, eine Imino-oder Acyliminogruppe bedeuten und deren pharmazeutisch verträgliche Säureadditionssalze besitzen antikonstriktorische Wirkung an peripheren und koronaren Gefäßen, hemmen ischämiebedingte Arrhythmien, besitzen broncholytische und antiasthmatische Wirkungen, erhöhen den koronaren und peripheren Blutfluß, hemmen die Thrombozytenaggregation, wirken antihyperglykämisch und vorteilhaft bei metabolischen Störungen, insbesondere solchen des Fettstoffwechsels. Sie können mit ihrer hohen pharmakologischen Wirkung als therapeutische Wirkstoffe und wertvolle Arzneimittel zur Behandlung und/oder Prophylaxe von Migräne, Angina pectoris, Myokardinfarkt, Asthma, koronaren und peripheren Durchblutungsstörungen, Morbus Raynaud, thromboembolischen Erkrankungen, Diabetes, Fettsucht und Glaukom sowie Lungen-, Bronchial-und Coronarerkrankungen, metabolischen Störungen und ischämiebedingten Arrhythmien verwendet werden. Die Verbindungen sind $alpha_2$-Antagonisten.

Gegenstand der Erfindung ist daher die Verwendung der Verbindungen der Formel I und deren pharmazeutisch verträglichen Säureadditionssalzen, mit der oben angegebenen Bedeutung von $R^1$, $R^2$, $R^3$, $R^4$ n und X zur Behandlung und/oder Prophylaxe von Migräne, Angina pectoris, Myokardinfarkt, Asthma, koronaren und peripheren Durchblutungsstörungen, beispielsweise Morbus Raynaud, thromboembolischen Erkrankungen, Diabetes, Fettsucht und Glaukom sowie Lungen-, Bronchial-und Coronarerkrankungen, metabolischen Störungen und ischämiebedingten Arrhythmien.

Gegenstand der Erfindung ist auch ein Verfahren zur Behandlung und/oder Prophylaxe von Migräne, Angina pectoris, Myokardinfarkt, Asthma, koronaren und peripheren Durchblutungsstörungen, beispielsweise Morbus Raynaud, thromboembolischen Erkrankungen, Diabetes, Fettsucht und Glaukom sowie Lungen-, Bronchial-und Coronarerkrankungen, metabolischen Störungen und ischämiebedingten Arrhythmien bei

Säugetieren sowie dem Menschen, indem diesen, insbesondere bei einer Erkrankung oder falls eine solche Behandlung oder Prophylaxe erforderlich ist, eine vorzugsweise nichttoxische Menge einer Verbindung der Formel I und deren Säureadditionssalzen mit der oben angegebenen Bedeutung von $R^1$, $R^2$, $R^3$, $R^4$ n und X verabreicht wird.

In einer weiteren Ausführungsform ist der Gegenstand der Erfindung auch die Verwendung der Verbindungen der Formel I und deren pharmazeutisch verträglichen Säureadditionssalzen, mit der oben angegebenen Bedeutung von $R^1$, $R^2$, $R^3$, $R^4$ n und X zur Herstellung von Arzneimitteln zur Behandlung und/oder Prophylaxe von Migräne, Angina pectoris, Myokardinfarkt, Asthma, koronaren und peripheren Durchblutungsstörungen, beispielsweise Morbus Raynaud, thromboembolischen Erkrankungen, Diabetes, Fettsucht und Glaukom sowie Lungen-, Bronchial-und Coronarerkrankungen, metabolischen Störungen und ischämiebedingten Arrhythmien.

Die vorliegende Erfindung bezieht sich auch auf Verbindungen der Formel I und deren pharmakologisch verträgliche Säureadditionssalze, mit der oben angegebenen Bedeutung von $R^1$, $R^2$, $R^3$, $R^4$ n und X zur Behandlung und/oder Prophylaxe von Migräne, Angina pectoris, Myokardinfarkt, Asthma, kornonaren und peripheren Durchblutungsstörungen, beispielsweise Morbus Raynaud, thromboembolischen Erkrankungen, Diabetes, Fettsucht und Glaukom sowie Lungen-, Bronchial-und Coronarerkrankungen, metabolischen Störungen und ischämiebedingten Arrhythmien.

Eine weitere Ausführungsform der Erfindung besteht in einer pharmazeutischen Zubereitung zur Verwendung bei der Behandlung und/oder Prophylaxe von Migräne, Angina pectoris, Myokardinfarkt, Asthma, koronaren und peripheren Durchblutungsstörungen, beispielsweise Morbus Raynaud, thromboembolischen Erkrankungen, Diabetes, Fettsucht und Glaukom sowie Lungen-, Bronchial und Coronarerkrankungen, metabolischen Störungen und ischämiebedingten Arrhythmien, die eine oder mehrere Verbindungen der Formel I und deren pharmazeutisch verträgliche Säureadditionssalze, mit der oben angegebenen Bedeutung von $R^1$, $R^2$, $R^3$, $R^4$ n und X und einen pharmazeutisch verträglichen Hilfsstoff oder Träger enthält.

Die Halogenatome $R^1$ und $R^2$ sind vorzugsweise Fluor, Chlor, Brom.

Die erfindungsgemäßen Alkylgruppen sowie die Alkylteile anderer Gruppen wie der Alkoxygruppen können geradkettig oder verzweigt sein und sind vorzugsweise Methyl-, Ethyl-, Propyl-und Butylgruppen.

Bevorzugte Acylgruppen sind die Formylgruppe und geradkettige oder verzweigte Alkylcarbonylgruppen bzw. Alkanoylgruppen und Aroylgruppen, vorzugsweise mit carbocyclischem Aryl. Vorzugsweise besitzen diese Gruppen jeweils 1 -7 Kohlenstoffatome, wobei die Phenylgruppe die bevorzugte Arylgruppe ist. Besonders bevorzugte Alkylcarbonylgruppen sind solche mit 1 -4 Kohlenstoffatomen, insbesondere die Formylgruppe, die Acetylgruppe und die Propionylgruppen. Bevorzugter Aroylrest ist der Benzoylrest.

Weiterhin bevorzugte Acylgruppen sind Cycloalkylcarbonylgruppen, vorzugsweise solche mit 4 -7 Kohlenstoffatomen. Bevorzugte Cycloalkylgruppen sind carbocyclische Cycloalkylgruppen wie die Cyclopropyl-, Cyclobutyl-, Cyclopentyl-und die Cyclohexylgruppe. Besonders bevorzugt werden Cyclopropyl-und Cyclohexylringe.

Die vorstehend definierten Acylgruppen können zusätzlich Substituenten besitzen.

Bevorzugte Substituenten der Acylgruppen, insbesondere der Formyl-und Alkylcarbonylgruppen, sind erstens geradkettige und verzweigte Alkoxygruppen, vorzugsweise solche mit 1 -4 Kohlenstoffatomen, besonders bevorzugt Methoxy-und Ethoxygruppen, zweitens Cycloalkylgruppen mit vorzugsweise 3 -6 Kohlenstoffatomen im Ringsystem wie vorstehend definiert, vorzugsweise mit Cyclopropyl-oder Cyclohexylring und drittens Arylgruppen, vorzugsweise carbocyclische Arylgruppen, insbesondere Phenylgruppen.

Besonders bevorzugte Acylgruppen sind wie vorstehend angegeben substituierte Acetylgruppen.

Eine Untergruppe von Verbindungen der Formel I, hervorgehoben durch die allgemeine Formel II

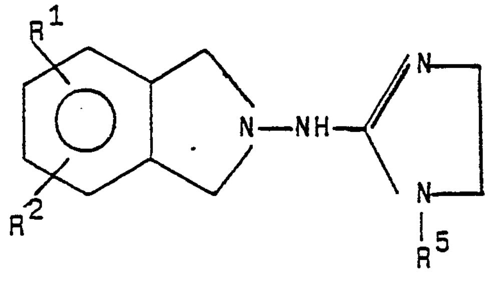

(II)

in der $R^1$ und $R^2$ die obengenannte Bedeutung haben und $R^5$ ein Wasserstoffatom oder eine substituierte

oder unsubstituierte Acylgruppe wie oben definiert bedeutet (X = Imino oder Acylimino), wird besonders bevorzugt.

Besonders bevorzugt sind Verbindungen der Formeln I und II mit $R^1$ = Wasserstoff, Halogen, Alkyl oder Alkoxy, insbesondere Wasserstoff oder Halogen, vorzugsweise Fluor oder Chlor, und $R^2$ = Wasserstoff, wobei die übrigen Reste die angegebene Bedeutung haben und Verbindungen der Formeln I und II in denen $R^1$ und $R^2$ nicht zugleich Wasserstoff bedeuten.

Für die Diabetesbehandlung besonders bevorzugte Verbindungen der Formeln I und II sind mit $R^1$ und $R^2$ disubstituiert, wobei die übrigen Reste die angegebene Bedeutung haben, so daß $R^1$ und $R^2$ dann nicht Wasserstoff bedeuten.

Die Substituenten $R^1$ und/oder $R^2$ der Formeln I und II befinden sich vorzugsweise in der 4-, oder 7-oder 4,7-Position, also jeweils in ortho-Stellung zu den beiden Ringen gemeinsamen Kohlenstoffatomen.

4-Halogenverbindungen der Formel II sind bevorzugt die 4-Fluor, 4-Chlor-oder 4-Bromverbindungen, insbesondere dann, wenn $R^2$ Wasserstoff bedeutet, aber die 4-Chlor-oder 4-Bromverbindungen.

Bevorzugte 4-Fluor-Verbindungen der Formel I, in der X Imino oder Acylimino und n = 2 bedeutet, bzw. der Formel II, sind solche, die zumindest noch einen weiteren, vom Wasserstoff verschiedenen Substituenten $R^2$, $R^3$ oder $R^4$ besitzen.

Gegenstand der Erfindung sind auch die neuen Verbindungen der Formeln I und II. Die Verbindungen der Formeln I und II sind neu, soweit sie nicht durch die vorstehend angeführten Patentanmeldungen vorweggenommen sind.

Die neuen Verbindungen der Formeln I und II werden für die Behandlung und/oder Prophylaxe von Diabetes bevorzugt.

Die folgenden Verbindungen der Formel I und deren Säureadditionssalze werden besonders bevorzugt:

2-(2-Imidazolin-2-ylamino)-isoindolin,
4-Fluor-2-(2-imidazolin-2-ylamino)-isoindolin,
4-Fluor-2-(1-acetyl-2-imidazolin-2-ylamino)-isoindolin,
4-Chlor-2-(2-imidazolin-2-ylamino)-isoindolin,
4,7-Dichlor-2-(2-imidazolin-2-ylamino)-isoindolin,
4,7-Dimethyl-2-(2-imidazolin-2-ylamino)-isoindolin,
4,7-Dimethoxy-2-(2-imidazolin-2-ylamino)-isoindolin,
4-Fluor-7-brom-2-(2-imidazolin-2-ylamino)-isoindolin,
1-Methyl-2-(2-imidazolin-2-ylamino)-isoindolin,

insbesondere aber

2-(2-Imidazolin-2-ylamino)-isoindolin,
4-Fluor-2-(2-imidazolin-2-ylamino)-isoindolin,
4-Fluor-2-(1-acetyl-2-imidazolin-2-ylamino)-isoindolin
und
4-Chlor-2-(2-imidazolin-2-ylamino)-isoindolin.

Die erfindungsgemäßen Verbindungen der Formeln I und II und die Säureadditionssalze können nach bekannten Methoden oder analog bekannten Methoden erhalten werden, beispielsweise mit den in den deutschen Offenlegungsschriften 28 16 627 und 29 05 501 und der europäischen Patentanmeldung 72 954 beschriebenen Verfahren. Die erfindungsgemäßen Acyliminoverbindungen sind auch durch Acylierung der entsprechenden Iminoverbindungen nach bekannten Acylierungsverfahren erhältlich.

Die Verbindungen können peroral oder parenteral verabreicht und auch lokal, beispielsweise als Augentropfen, angewendet werden.

Zur Behandlung der vorstehend aufgeführten Krankenheiten bei Menschen können die Verbindungen der Formel I und deren Säureadditionssalze einmal oder mehrmals täglich verabreicht werden. Die Tagesdosis liegt bei oraler Anwendung vorzugsweise bei 0,1 mg bis 10 g, insbesondere bei 1 bis 1000 mg und besonders bevorzugt bei 10 bis 100 mg. Es ist zweckmäßig, die Tagesdosis individuell abzustimmen, weil sie von der Rezeptorenempfindlichkeit des Patienten abhängt. Zweckmäßigerweise wird die Behandlung mit niederen Dosen begonnen und dann gesteigert.

Zur Behandlung des erhöhten Augeninnendrucks werden vorzugsweise 0,01 bis 20 %ige isotone Lösungen in Dosierungen von einem oder mehreren Tropfen ein oder mehrmals täglich lokal am Auge angewendet.

Die Verbindungen gemäß der Erfindung können mit üblichen pharmazeutisch verträglichen Hilfsstoffen oder Trägern oder auch anderen Substanzen vermischt und beispielsweise zu oralen, sublingualen, parenteralen, topischen, rektalen Arzneiformen und zu Injektionslösungen oder -suspensionen sowie Tropflösungen zum Aufbringen auf Schleimhäute verarbeitet werden. Sie können oral in Form von Tabletten, Dragees, Sirupen, Pulvern, Suspensionen, Hart-und Weichgelatinekapseln und Flüssigkeiten oder parenteral in Form von Lösungen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerte Verdünnungsmittel wie Calciumcarbonat, Natriumcarbonat, Lactose und Talk, Granulierungsmittel und Mittel, die den Zerfall der Tabletten bei oraler Verabreichung fördern wie Stärke oder Alginsäure, Bindemittel wie Stärke oder Gelatine, Gleitmittel wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Die Tabletten können nach bekannten Arbeitsweisen überzogen werden, um den Zerfall und die Resorption im Magen-Darm-Trakt zu verzögern oder auch den Zutritt von Magensaft zu verhindern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermittel wie Methylcellulose, Tragacanth oder Natriumalginat, Netzmittel wie Lecithin,
Polyoxyethylenstearat und
Polyoxyethylensorbitanmonooleat und Konservierungsmittel wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 99%, enthalten, wobei der Rest ein Trägerstoff oder Zusatzstoff ist. Im Hinblick auf die Herstellung und Verabreichung werden feste Präparete wie Tabletten und Kapseln bevorzugt. Vorzugsweise enthalten die Präparate den Wirkstoff in einer Menge von 0,1 mg bis 2 g, insbesondere 0,1 mg bis 0,1 g.

Beispiel 1

Tabletten, die die im folgenden genannten Bestandteile enthalten, lassen sich in bekannter Weise herstellen. Sie können zur Behandlung von Migräne oder Angina pectoris oder Myokardinfarkt oder Asthma oder koronaren und peripheren Durchblutungsstörungen oder Morbus Raynaud oder thromboembolischen Erkrankungen oder Diabetes oder Fettsucht oder Glaukom sowie Lungen-, Bronchial-und Coronarerkrankungen, metabolischen Störungen und ischämiebedingten Arrhythmien einmal oder mehrmals täglich angewendet werden.

| | | | |
|---|---|---|---|
| 4 Chlor-2-(2-imidazolin-2-ylamino)-isoindolin | 1 mg | 50 mg | 100 mg |
| Lactose | 75 mg | 75 mg | 75 mg |
| Maisstärke | 10 mg | 10 mg | 10 mg |
| Mikrokristalline Cellulose | 8 mg | 8 mg | 8 mg |
| Polyvinylpyrrolidon | 1 mg | 1 mg | 1 mg |
| Magnesiumstearat | 0,5 mg | 0,5 mg | 0,5 mg |
| hochdisperses Siliziumdioxid | 0,5 mg | 0,5 mg | 0,5 mg |

Beispiel 2

Es wird wie im Beispiel 1 verfahren, aber anstelle von 4-Chlor-2-(2-imidazolin-2-ylamino)-isoindolin wird 2-(2-Imidazolin-2-ylamino)-isoindolin verwendet.

Beispiel 3

Herstellung von Augentropfen

Augentropfen, die die folgenden Bestandteile enthalten, lassen sich in bekannter Weise herstellen. Sie können in einer Dosis von ein bis zwei Tropfen pro Auge ein bis zweimal täglich, vorzugsweise ein Tropfen zweimal täglich, verwendet werden:

4-Chlor-2-(2-imidazolin-2-ylamino)-isoindolin 0,5 g

isotone wäßrige Lösung (Ringerlösung) ad 100 ml

Beispiel 4

Es wird wie im Beispiel 1 verfahren, aber anstelle von 4-Chlor-2-(2-imidazolin-2-ylamino)-isoindolin wird 4-Fluor-2-(2-imidazolin-2-ylamino)-isoindolin verwendet.

Beispiel 5

Es wird wie im Beispiel 3 verfahren, aber anstelle von 4-Chlor-2-(2-imidazolin-2-ylamino)-isoindolin wird 4-Fluor-2-(2-imidazolin-2-ylamino)-isoindolin verwendet.

**Ansprüche**

1. Verwendung von Verbindungen der Formel I

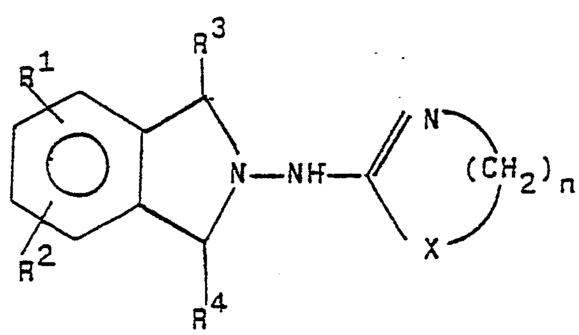

(I)

in der

$R^1$ und $R^2$, die gleich oder verschieden sein können, ein Wasserstoff-oder Halogenatom, eine Alkylgruppe oder Alkoxygruppe mit jeweils 1 -4 Kohlenstoffatomen, $R^3$ und $R^4$, die gleich oder verschieden sein können, ein Wasserstoffatom oder eine Alkylgruppe mit 1 -4 Kohlenstoffatomen,
n die Zahl 2 oder 3 und
X ein Sauerstoff-oder Schwefelatom, eine Methylengruppe, eine Imino-oder Acyliminogruppe bedeuten und deren pharmazeutisch verträglichen Säureadditionssalzen zur Herstellung von Arzneimitteln zur Behandlung und/oder Prophylaxe von Migräne, Angina pectoris, Myokardinfarkt, Asthma, koronaren und peripheren Durchblutungsstörungen, Morbus Raynaud, thromboembolischen Erkrankungen, Diabetes, Fettsucht und

Glaukom sowie Lungen-, Bronchial-und Coronarerkrankungen, metabolischen Störungen und ischämiebedingten Arrhythmien.

2. Verwendung von Verbindungen der Formel II

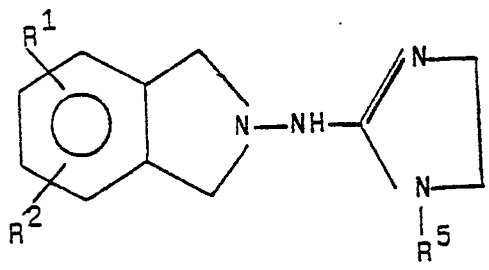

(II)

in der R' und R² die obengenannte Bedeutung haben und $R^5$ ein Wasserstoffatom oder eine Acylgruppe bedeutet, gemäß Anspruch 1.

3. Verwendung von Verbindungen der Formeln I oder II, worin R' und/oder R² sich in 4-und/oder 7-Position befinden, gemäß Anspruch 1.

4. Verwendung von
2-(2-Imidazolin-2-ylamino)-isoindolin oder
4-Fluor-2-(2-imidazolin-2-ylamino)-isoindolin oder
4-Fluor-2-(1-acetyl-2-imidazolin-2-ylamino)-isoindolin
oder
4-Chlor-2-(2-imidazolin-2-ylamino)-isoindolin gemäß Anspruch 1.

5. Pharmazeutische Zubereitung zur Verwendung bei der Behandlung und/oder Prophylaxe von Migräne, Angina pectoris, Myokardinfarkt, Asthma, koronaren und peripheren Durchblutungsstörungen, Morbus Raynaud, thromboembolischen Erkrankungen, Diabetes, Fettsucht und Glaukom sowie Lungen-, Bronchial-oder Coronarerkrankungen, metabolischen Störungen und ischämiebedingten Arrhythmien, die eine oder mehrere Verbindungen der Formel I gemäß Anspruch 1 und deren pharmazeutisch verträgliche Säureadditionssalze und einen pharmazeutisch verträglichen Hilfsstoff oder Träger enthält.

6. Verwendung der Verbindungen der Formel I gemäß Anspruch 1 und deren pharmazeutisch verträgliche Säureadditionssalze zur Behandlung und/oder Prophylaxe von Migräne, Angina pectoris, Myokardinfarkt, Asthma, koronaren und peripheren Durchblutungsstörungen, Morbus Raynaud, thromboembolischen Erkrankungen, Diabetes, Fettsucht und Glaukom sowie Lungen-, Bronchial-und Coronarerkrankungen, metabolischen Störungen und ischämiebedingten Arrhythmien.